# EUROPEAN PATENT SPECIFICATION

(11) **EP 1 587 562 B1**
(45) Date of publication and mention of the grant of the patent: **18.04.2007**
(21) Application number: 04703033.3
(22) Date of filing: 16.01.2004
(51) Int. Cl.: A61M 5/20, A61M 5/315

(54) **PEN STYLE LIQUID DISPENSER**
STIFTARTIGER FLÜSSIGKEITSSPENDER
DISTRIBUTEUR DE LIQUIDE DE TYPE STYLO

(30) Priority: 17.01.2003 US 441016 P
(43) Date of publication of application: 26.10.2005
(73) Proprietor: DENTSPLY INTERNATIONAL, INC., York, PA 17405-0872 (US)
(72) Inventor: SCHIFF, David, R., Highland Park, NJ 08904 (US); FORT, Tucker, New York, NY 10014 (US)
(74) Representative: Hartz, Nikolai
(86) International application number: PCT/US2004/001220
(87) International publication number: WO 2004/064900

(56) References cited:
- FR-A- 1 014 881
- US-A- 2 221 739
- US-A- 2 309 502

## Description

### TECHNICAL FIELD

The present invention is a hand-held device useful for dispensing liquid materials including gels. It has a resettable ratchet mechanism and a release lever, such when the lever is released, a piston is biased toward a delivery nozzle, such that an amount of material is urged to flow toward and ultimately out of the nozzle. A ratchet mechanism is used to selectively stop the biased movement of the piston. The device is configured and held in a style like a pen. Such devices are well known in the art, for example US-A-2 221 739.

### BACKGROUND OF THE INVENTION

It is known to employ pen style devices for the injection of medications and the like. It is also known to employ a ratchet mechanism such that an amount of material exiting from the device is controlled by operation of the ratchet to move a piston. Multiple physical movement by the operator are often required to make a single dose. This often causes hesitant and non-stable or "jerky" movements on the part of the operator and discomfort to the patient. A need exists for a selectively constant flow hand-held fluid delivery device.

### SUMMARY OF THE INVENTION

The present invention provides a hand-held device useful for dispensing liquid materials including gels. By "liquid" therefore it is meant and flowing material regardless of viscosity, although the present invention has particular application to dental restorative, prophylactic, medicament and pharmaceutical materials. The present invention has a resettable ratchet mechanism and a release lever, such when the lever is released, a piston is biased toward a delivery tip, such that an amount of material is urged to flow toward and ultimately out of the tip. A ratchet mechanism is used to selectively stop the biased movement of the piston. The device is configured and held in a style like a pen whereby said device comprises a pen-like housing configured in two parts to receive the delivery tip and an ampoule containing dental material to be dispensed.

### BRIEF DESCRIPTION OF THE DRAWINGS

Fig.1 is a perspective view of a fluid delivery device according to the present invention.

Fig.2 is an exploded view showing the internal components of a preferred version of the device of Fig. 1.

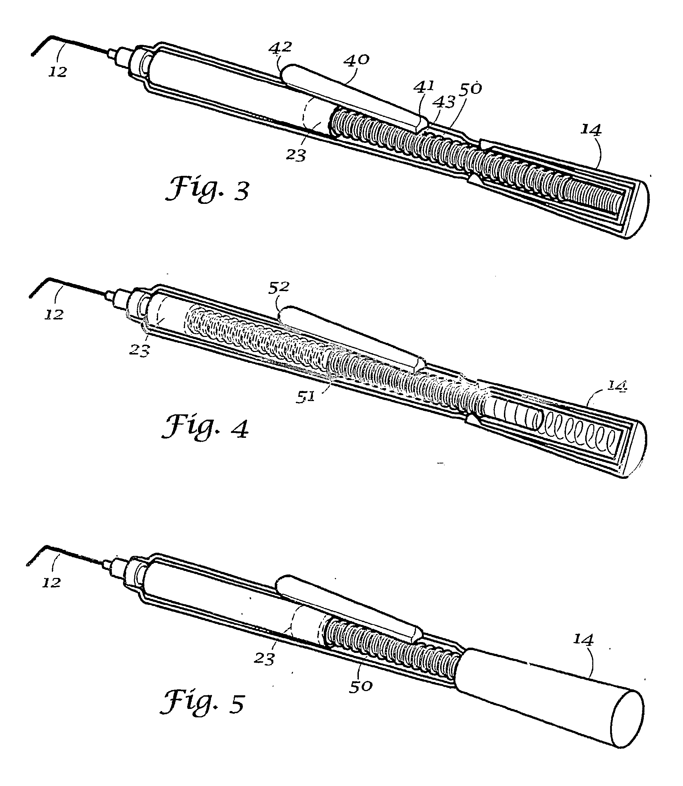

Fig. 3 is a sectional, side-perspective view of the device of Fig. 1, showing the device prior to delivery of the fluid contained therein.

Fig. 4 is a. sectional, side-perspective view of the device of Fig. 1, showing the device during delivery of the fluid contained therein, and stopped at some mid-point of such delivery.

Fig. 5 is a sectional, side-perspective view of the device of Fig. 1, showing the device in its reset position after delivery of the material.

### PREFERRED EMBODIMENTS FOR CARRYING OUT THE INVENTION

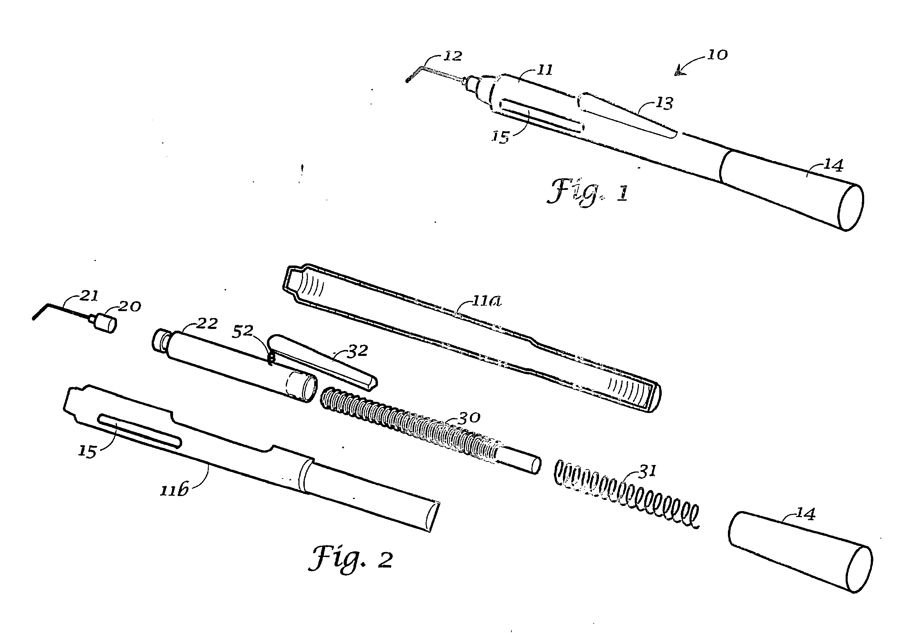

Fig. 1 shows an external view of a liquid delivery device 10 according to the present invention. It includes a preferably pen-style housing 11, a delivery tip 12, a release lever 13 and a reset handle 14. Housing 11 may include a site window 15 so that an operator can visually see the amount of material contained within device 10.

Fig. 2 shows an exploded view of a preferred embodiment of a device 10 according to the invention. Housing 11 may be configured in two parts 11 and 11b. Delivery tip 12 may be any conventional tip, and may be configured for delivery of any fluid material. It may include a hub 20 and a conduit 21, and conduit 21 may even be a hypodermic needle. Although not required, conduit 21 may be formed from a bendable metal or a plastic material. Hub 20 is employed to attach delivery tip 12 to an ampoule 22 by any conventional means. Ampoule 22 is also preferably of conventional design, and may include any fluid material such as a dental material. As is known, ampoule 22 preferably has therein a follower 23. Housing 11 and if employed, housing parts 11a and 11b are preferably configured to receive ampoule 22 and delivery tip 12.

A piston 30 is also received within housing 11 such that it is axially juxtaposed with ampoule 22 at an end of ampoule 22 distal to delivery tip 12. A bias device such as spring 31 biases piston 30 towards ampoule 22, such that physical contact between piston 30 and follower 23, causes follower 23 to receive the same force exerted by spring 31, thereby moving follower 23 toward the end of ampoule 22 proximal to delivery tip 12. Thus, material contained within ampoule 22 is caused to flow toward delivery tip 12 for delivery therefrom.

A ratchet lever 40 is provided such that it is at least partially received through housing 11 at one end 41, and is exposed on the external side of housing 11 at end 42. Received end 41 of lever 40 is also preferably provided with a surface complementary to the surfaces of a plurality of teeth 50 carried on the external surface of piston 30. By being so complementary, end 41 of lever 40 is caused to physically engage one of the plurality of teeth 50, to thereby stop movement of piston 30 when so physically engaged. When movement of piston 30 is desired, end 42 of lever 4.0 is depressed toward housing 11, thereby disengaging end 41 from its physical contact with one or more teeth 50, and thereby allowing substantially free movement of piston 30 toward follower 23. Piston 30 will continue such movement until either the ampoule 22 is emptied and the follower engages the proximal end of ampoule 22, or until the operator releases end 42 of lever 4.0, thereby allowing end 41 of lever 40 to again physically contact and engage a tooth 50, stopping such movement. The operator can then selectively dispense material by a single depression of lever 40, as well as stopping and starting such delivery. During delivery however, the material will flow due to the biasing force of spring 31 without further need of depressing any lever or release mechanism.

Lever 40 is preferably configured with a pivot fulcrum 51, but may also include a pivot pin or the like. Further, it is preferred to employ a lever spring 52 positioned between housing 11 and end 42 of lever 4.0, such that unless end 42 is depressed during delivery, end 42 is biased away from housing 11 thereby causing end 41 of lever 40 to be in its physically engaged position with at least one tooth 50.

Reset handle 14 is provided so that it is affixed or operatively affixed to piston 30, such that manipulating handle 14 by pulling it away from delivery tip 12 will cause piston 30 to move away from delivery tip 12 and will also cause spring 31 to be compressed. With a charged ampoule 22 placed within housing 11, the device 10 is then in position to deliver the material contained within ampoule 12.

It should be apparent that a fluid delivery device as described will carry out the function of the invention. The device has been exemplified by the present description and attached drawings without attempting to show all of the variations and embodiments within the scope thereof.

## Claims

1. A hand-held device (10) useful for dispensing a liquid material in a style like a pen, comprising:
a housing (11) having a resettable ratchet mechanism operatively connected to a biased piston (30); said ratchet mechanism including a release lever (13) normally preventing movement of said piston (30) until released,
wherein when said lever is released, said piston is biased toward a delivery tip (12), such that an amount of the material is urged to flow toward the delivery tip (12) **characterized by** a pen-like housing (11) configured in two parts (11 a, 11 b) to receive the delivery tip (12) and an ampoule (22) containing dental material to be dispensed.

2. A dispensing device as in claim 1, wherein said piston comprises a plurality of ratchet teeth operatively interacting with said lever.

3. A dispensing device as in claim 2, wherein said lever is pivotally mounted and is biased such that it is normally in physical contact with at least one of said plurality of ratchet teeth until said lever is manually pivoted out of said physical contact.

## Patentansprüche

1. Stiftartige Handvorrichtung (10) zur Abgabe eines flüssigen Materials, umfassend:
ein Gehäuse (11), das einen mit einem vorgespannten Kolben (30) operativ verbundenen rückstellbaren Sperrmechanismus aufweist; der Sperrmechanismus einen Auslösehebel (13) umfasst, der im Normalfall die Bewegung des Kolbens (30) bis zur Auslösung verhindert, wobei, wenn der Hebel ausgelöst wird, der Kolben gegen eine Abgabespitze (12) so gedrückt wird, dass eine Menge des Materials dazu veranlasst wird, gegen die Abgabespitze (12) zu fließen, **gekennzeichnet durch** ein schreibfederartiges Gehäuse (11), das in zwei Teilen (11a, 11b) konfiguriert ist, um die Abgabespitze (12) und eine Ampulle (22), die das abzugebende Dentalmaterial enthält, aufzunehmen.

2. Abgabevorrichtung nach Anspruch 1, worin der Kolben eine Mehrzahl von mit dem Hebel operativ in Wechselwirkung stehenden Sperrzähnen aufweist.

3. Abgabevorrichtung nach Anspruch 2, worin der Hebel schwenkbar befestigt und so eingestellt ist, dass er im Normalzustand in physikalischem Kontakt mit mindestens einem der Mehrzahl von Sperrzähnen steht, bis der Hebel manuell aus diesem physikalischen Kontakt geschwenkt wird.

## Revendications

1. Dispositif tenu à la main (10) servant à distribuer une matière liquide à la manière d'un stylo, comprenant :
un logement (11) comportant un mécanisme d'encliquetage à réarmement automatique, fonctionnellement relié à un piston sollicité (30) ; ledit mécanisme d'encliquetage incluant un levier de débrayage (13) empêchant normalement le mouvement dudit piston (30) jusqu'à débrayage, dans lequel lorsque ledit levier est débrayé, ledit piston est sollicité vers une pointe de distribution (12), de manière qu'une quantité de la matière soit amenée à s'écouler vers la pointe de distribution (12), **caractérisé en ce qu'**un logement analogue à un stylo (11) est configuré en deux parties (11a, 11b) pour recevoir la pointe de distribution (12) et une ampoule (22) contenant la matière dentaire devant être distribuée.

2. Dispositif distributeur selon la revendication 1, dans lequel ledit piston comprend une pluralité de dents d'encliquetage en interaction fonctionnelle avec ledit levier.

3. Dispositif distributeur selon la revendication 2, dans lequel ledit levier est monté pivotant et est sollicité de manière à être normalement en contact physique avec au moins une dent de ladite pluralité de dents d'encliquetage jusqu'à ce que ledit levier soit amené à pivoter manuellement hors dudit contact physique.
